# EUROPEAN PATENT APPLICATION

(11) **EP 4 763 135 A1**
(43) Date of publication of application: **24.06.2026**
(21) Application number: 24853080.0
(22) Date of filing: 01.07.2024
(51) Int. Cl.: A61D 19/04, A61D 19/02, A61B 17/435, A61B 10/02

(54) **DOUBLE-LUMEN OOCYTE ASPIRATION NEEDLE AND USE THEREOF**

(30) Priority: 17.08.2023 BR 102023016625
(71) Applicant: WTA - WATANABE TECNOLOGIA APLICADA LTDA, SP, CEP 14140-000 Cravinhos (BR)
(72) Inventor: YOSHIME WATANABE, Osnir, 14140-000 Cravinhos (BR); ORDOZ BARISSA, Adriano, 14140000 Cravinhos (BR)
(74) Representative: Algemeen Octrooi- en Merkenbureau B.V.
(86) International application number: PCT/BR2024/050283
(87) International publication number: WO 2025/035194

(57) **Abstract**

The present invention relates to a double-lumen oocyte aspiration needle and use thereof in the collection of oocytes from the ovaries of animals undergoing assisted reproduction procedures.

## Description

### FIELD OF THE INVENTION

The present invention is located in the fields of Animal Science, Veterinary Medicine, Biology and Engineering and is intended for a double lumen oocyte aspiration needle and its use in the collection of oocytes in ovaries of animals in assisted reproduction procedure.

### BACKGROUND OF THE INVENTION AND PRIOR ART

Assisted reproduction procedures in animals have become widely accepted and applied because they facilitate or enhance the encounter between male and female gametes so that the fertilization occurs, which can be applied in the animal *in vivo,* as in laboratory *in vitro,* thus generating better reproductive efficiency and genetic improvement in agricultural practice.

In the process of aspiration or retrieval of oocytes, a needle is introduced which, in contact with the ovary, pierces a follicle in which the oocytes are collected by aspiration through a hose connected to the opposite end of the needle bevel and integrated into a container comprising a cap and a test tube that communicates with a vacuum source. As soon as it is sucked by the needle cannula, the oocyte passes through a hose, and is later deposited in a test tube.

The double lumen oocyte aspiration needle comprises an external needle (responsible for piercing the animal's follicle) and an internal cannula (which aspirates the desired material). The space between the diameter of the needle and the internal cannula forms a passage for the application of fluid, used to wash the perforated region. The inner cannula is connected to a vacuum system for aspiration, and the space between the needle and the inner cannula is connected to a second hose into which the fluid is applied through a syringe or float. This hydraulic system utilizes a properly sealed Y-type conductor to ensure that the fluid is directed to the desired end (needle tip). In this mentioned model, the Y-system ends up being too large and, therefore, it is necessary to be on the outside of an aspiration guide (adapter used with ultrasound and passage channel for the needle). Thus, it is necessary to compensate for the stability of the system by using a longer needle.

State-of-the-art models of double lumen oocyte aspiration needles are known, which are composed of a long needle, connected to a two-way chamber at the opposite end of the needle bevel, and one of the ways contains a hose where fluids for irrigation are applied, and on the other side a cannula with a relatively smaller diameter than the internal diameter of the needle, which runs along the entire length until close to the needle bevel, this cannula is also connected to a hose, at its opposite end to the needle bevel, which leads to the container composed of the cap and test tube. A seal is required in this two-way chamber to prevent fluid leakage and ensure that oocytes follow the flow of the process. Thus, in the oocyte aspiration or retrieval procedure, the injected fluid helps to detach the oocytes from the follicles and promotes better fluidity in aspiration. For a better constructive understanding, a double lumen oocyte aspiration needle basically comprises a smaller channel (internal cannula) within a larger channel (needle lumen). A fluid is injected into the space created between both channels, helping the aspiration of oocytes through the smaller canal (internal cannula). The two-way chamber connected at the opposite end of the needle bevel usually has a "Y" or "T" shape and has a fluid application hose connection and a hose connection that is directly connected to the internal cannula, isolated from the entire chamber that directs the fluid into the space between the channels. In this way, two routes are provided, one for injection and the other for aspiration, in which the aspirated fluid is expelled at the end near the bevel and then aspirated through the internal cannula along with the material to be collected.

In order to be able to perform this procedure of oocyte collection or retrieval by aspiration process in large animals such as horses and cattle, it is necessary to visually monitor the process through ultrasound, which is performed intravaginally in the animal by means of an adapter device (aspiration guide), which accommodates the transducer and protects the entire system with the needle in a cavity in the device itself, thus making it possible to introduce a needle and transducer through the vaginal canal without causing injury to the animal. In general, these adapter devices (aspiration guides) are made anatomically and with the smallest possible size, in order to avoid discomfort for the animal, resulting in a cavity with limited dimensions for the accommodation of the needle in said device.

The international publication WO 93/07917 refers to a double-lumen oocyte collecting device. Said oocyte collector device includes parallel cannulas that extend axially outward from a proximal terminal of the connection/hub. The device also provides a finger pressure on the connection/hub. Such a depression located in the connection/hub corresponds directly to an elongated beveled point of a cannula extending from the distal terminal of the connection/hub. The parallel single cannulas and finger depression on the connection/hub allow medical professionals to uniformly rotate and precisely orient the beveled tip of the device to perforate a follicle in an ovary with minimal trauma prior to oocyte collection.

The international publication WO 2009/040493 refers to a sampling needle, in particular being a needle suitable for obtaining oocytes from humans and animals, and the needle comprises a first tubular region in fluid communication with a second tubular region, the first region comprising a guide terminal for insertion into a human or animal and a second tubular region comprising a terminal mobile region for communication fluidic, in terms of receiving a fluid, and the first tubular region has an external diameter that is less than the internal diameter of the secondary tubular region. Said prior art document also refers to a method of obtaining a sample, in particular a human or animal oocyte.

The US patent application 2008/091119 AI refers to a device and method for verifying the characteristics of the follicular fluid *in vivo,* comprising an aspiration needle for obtaining oocytes having one or more sensors arranged in the external cannula or incorporated in its external cannula wall for sensing and measuring the characteristics of the follicular fluid constituents, such as pH level and oxygen concentration. Sensors are micro sensors that communicate via physical connection or wireless connection (*wireless*) with a meter and display. The measurements are recorded and optionally printed for future review and selection of the most viable oocytes. This method involves inserting an aspiration needle into the ovary with one or more sensors. The needle is guided to a first follicle and inserted into a first follicle. Follicular fluid characteristics are monitored and measured as displayed on the meter and recorded for future reference and selection.

The international publication WO 2010/054660 AI refers to a double cannula system for obtaining oocytes. The double cannula system has an inner cannula and an outer cannula, with the inner cannula operating freely within the outer cannula. In addition, the inner cannula can be removed from the outer cannula, and both cannulas have a tip that is pointed and beveled. The tip of the inner cannula of the dual cannula system is at the front when introduced into an individual and the inner cannula is used to locate one or more parts of anesthesia when the inner cannula is procedurally introduced into an individual. The tip of the outer cannula runs behind the tip of the inner cannula. The inner cannula is removed before cells and/or fluid from the body are obtained through the outer cannula.

It is known that, in most models of double lumen oocyte aspiration needles, a typical construction comprises a long needle of approximately 500 mm, for movement within the cavity of these adapter devices (aspiration guides), with an internal cannula joined in a chamber, which directs the fluid between the gap generated by a gap in the cannula and the internal part of the needle, in which it also promotes a seal on the end of the internal cannula where a hose is attached for the collection of the aspirated material.

Due to the anatomy of large animals, these adapter devices (aspiration guides) are far away, which requires a needle with lengths in the range of 500 mm, causing a negative effect on the act of aspiration, as it subjects the oocyte to a long distance through a metabolic area with a high coefficient of friction, which causes wear of these cells.

The company Planer Limited sells a double lumen needle, called "Kitazato", which allows aspirating oocytes and releasing the follicle in the same procedure, through different channels. The double lumen needle has two channels inside, one of which is a central channel with a larger diameter and allows the aspiration of oocytes and follicular fluid. There is also a second channel that allows the release of the sterilizing agent for the cleansing of the follicle. The double channel needle marketed by this company is also a needle in which its construction requires a long needle cannula and internal metal tubing.

The company Vitrolife Group sells the product called "Sense^{™} Single and Double Lumen", which has a 16G type needle body and a 19G type needle tip. It is a double channel needle that stands out for the staggering in the extension of the needle cannula, which decreases the diameter in the bevel region in order to provide a small area in diameter in the pierced region, but always following the conventional state-of-the-art construction of long conventional double flow needles.

The company Cook Medical^{®} sells a product called "Cook^{®} EchoTip^{®} Double Lumen Aspiration Needle", which is used to aspirate and release oocytes from ovarian follicles. It is a conventional double lumen needle, with the differential of having micro markings in the bevel region that better reflect the ultrasound, thus improving the visualization and location of the needle in the aspiration process.

It is also evident, from the state of the art, that the technical problem still to be solved is the reduction of lesions and loss of oocytes in quality and/or viability, due to the friction of the oocytes on the internal walls of the piping of the aspiration devices.

Taking into account that aspiration needles are single-use and disposable items, devices containing long needles with average measures of 500 mm in length and with an internal metal pipe become a considerable problem in the disposal of these materials, directly impacting the issue of waste generation to be later incinerated, also impacting the environmental issue.

In view of all the above, the Filing Applicant has developed a double lumen oocyte aspiration needle that presents a compact constructive configuration, presenting a bifurcated chamber, ensuring a watertight sealing of the hydraulic system, as well as a reduced aspiration needle, promoting the aspiration of a greater quantity and quality of viable oocytes, since the aspirated oocytes do not have any type of contact with the metal parts of high coefficient of device friction.

### DESCRIPTION OF THE DRAWINGS

Figure 1 illustrates a perspective view of the compact design of the double-lumen oocyte aspiration needle of the present invention.
Figure 2 illustrates a cross-sectional lateral representation of the compact design of the double-lumen oocyte aspiration needle of the invention.
Figure 3 illustrates a lateral representation of the compact construction configuration of the invention with enlarged detail in section.

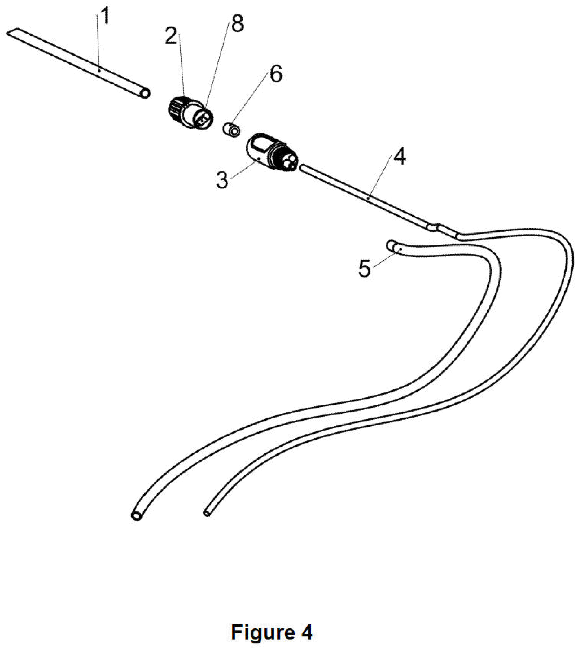

Figure 4 illustrates a burst view of the components in rear perspective of the needle of the present invention.

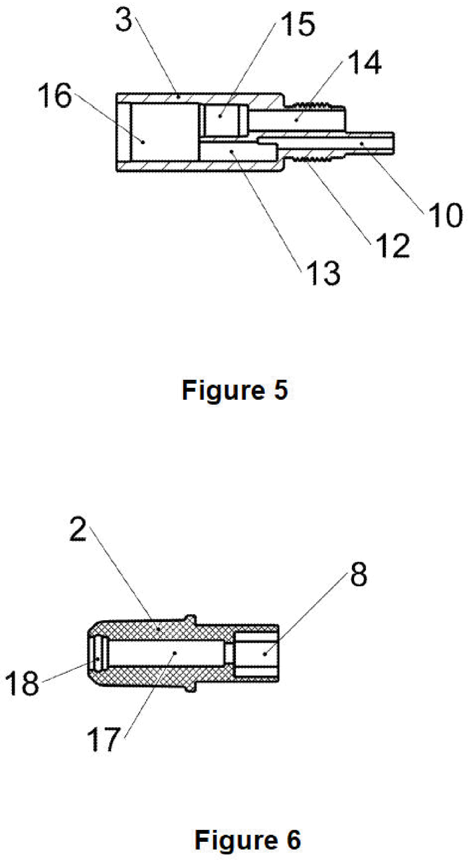

Figure 5 illustrates a lateral cross-sectional representation of the rear base component of the compact design of this invention.
Figure 6 illustrates a lateral sectional representation of the front base component of the compact design of this invention.

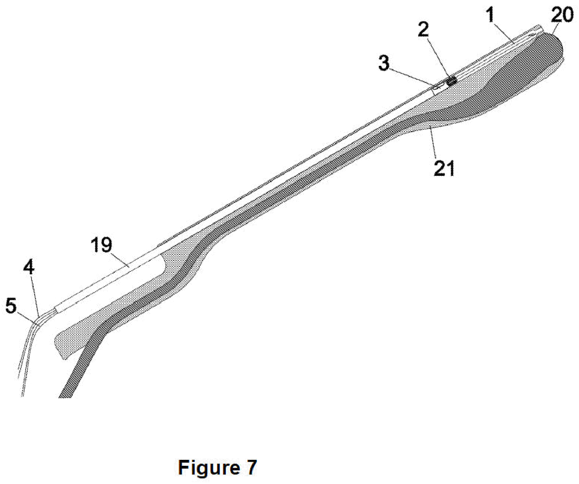

Figure 7 illustrates a cross-sectional lateral representation of the compact design of the double-lumen oocyte aspiration needle of the present invention mounted on a device adapter (aspiration guide).

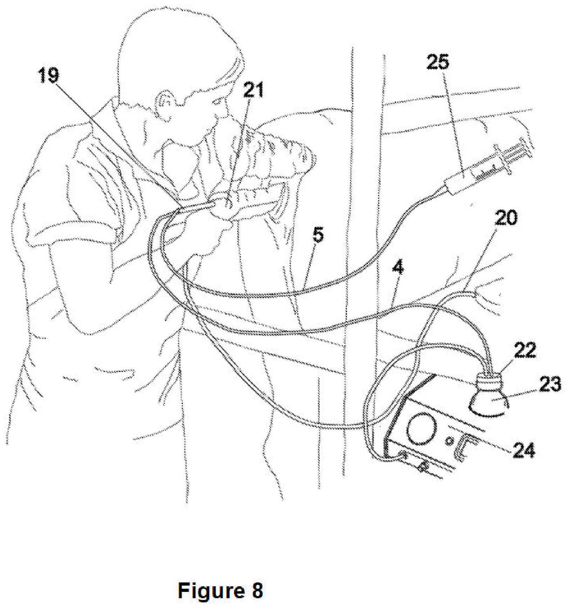

Figure 8 illustrates a linear drawing representation of a veterinarian performing oocyte aspiration using the dual-lumen aspiration needle of this invention.
Figure 9 shows a photograph taken on a Nikon SMZ800N stereoscope using a PrimeCam INTERVISION SMZ12 camera, which shows the comparison between oocytes aspirated with a double lumen oocyte aspiration needle of the present invention in group A and aspirated by a 500 mm long cannula double lumen aspiration needle, of the state of the art, in group B.

### DETAILED DESCRIPTION OF THE INVENTION

In some alternatives of oocyte aspiration needles with double lumen, a bifurcated chamber is provided in a reduced way in order to accommodate the entire set within the cavity of these adapter devices (aspiration guides), thus allowing the reduction of the length of the needle and the internal aspiration cannula, which would cause the smaller oocyte to have friction with its cells, due to the smaller area of contact with the metal cannula until it reaches the hose that is usually made of polymers with low coefficient of friction. These needle models should be used with the help of a rod screwed or glued to this bifurcated chamber, necessary for its movement in the act of perforating the follicles. However, one of the main problems encountered in double lumen oocyte aspiration needles with reduced bifurcated chamber is the construction difficulty, since it is a system that requires a good seal, due to the pressure applied in the act of injecting the fluid. This serious sealing made by some type of glue that, hypothetically, would not guarantee good repeatability in production and would generate leaks in the system, not guaranteeing a good flow of fluid for washing of the follicle. Another serious option is the manufacture by overinjection of thermoplastic on the internal metal cannula and glued or screwed in the bifurcated chamber, which would give the production process good repeatability, but higher cost, the problem of the metal cannula with the high coefficient of friction remaining.

The present invention refers to a double lumen oocyte aspiration needle and its use in the collection of oocytes in ovaries of animals undergoing assisted reproduction procedures.

In a first embodiment, the patent application provides a double lumen oocyte aspiration needle that has a bifurcated chamber, ensuring a watertight seal of the hydraulic system, as well as a 12G (gauges) aspiration needle, 3 mm in diameter and with a total length of less than 110 mm, on average 5 times less than traditional needles of the state of the art, through which a pipe with a low coefficient of friction passes internally, promoting the aspiration of a greater quantity and quality of viable oocytes, since the aspirated oocytes do not have any type of contact with the metal parts of said device.

The compact constructive configuration of the aspiration needle disclosed in the present invention, due to its watertightness, prevents leaks and loss of pressure during aspiration, which directly affect the production of viable oocytes in animals.

The present invention also proposes the replacement of the internal channel (metal cannula used in the state of the art) by a hose made of thermoplastic material with a low static friction coefficient of about 0.05 p, which is fixed and insulated, by compression, by means of a silicone rubber sealing tube, together with an adhesion material, the entire internal watertight hydraulic system consists of the bifurcated chamber and needle of reduced sizes. Such a sealing tube accommodated in the cavity properly designed to create a sufficient grip around said hose in order to fasten it, also creates a watertight seal of the bifurcated chamber composed of the front base piece and the rear base piece, generating a better direction for the fluid injected in the follicle washes.

In addition, the present invention provides an oocyte aspiration needle with double lumen that, in addition to being compact, is totally disposable, without the need to assemble parts in the field, which also reduces the contamination rates at the time of production of animal embryos, such as cattle, horses, pigs, among others.

The compact design of the double lumen oocyte aspiration needle of the present invention consists of an aspiration hose (4), consisting of a thermoplastic material with a low coefficient of friction, inserted internally along the entire watertight hydraulic system, i.e. from the internal space of the needle (1) connected to a bifurcated chamber composed of the front base piece (2) which, in turn, is attached to the rear base piece (3) to a rod (19) that acts as an extension for moving the compact design of the oocyte aspiration needle with double lumen inside an adapter device "aspiration guide" (21).

In general, the compact construction configuration of the double lumen oocyte aspiration needle of this invention comprises a needle (1) fitted to the duct (17) and joined in the fastening region (7) with adhesion material, in particular, epoxy glue wrapped to the housing (18) internal to the front base piece (2), and the duct (17) limits the depth of the needle (1) to be fitted. The housing (18) is a concave profile that prevents the fastening area (7) from detaching with adhesion material, in particular epoxy glue, once solidified.

In the rear base piece (3), the aspiration hose (4) is passed through the channel (14) and is held down by the silicone rubber sealing tube (6) housed in a subsequent cavity (15) aligned with the channel (14). The aspiration hose (4) is slightly curved into a relief and communication space (8), through which it is routed internally to the needle (1) to close to the bevel (11), leaving a spacing (9) between the inner walls of the needle (1) and the outer surface of the aspiration hose (4).

The relief and communication space (8) creates direct communication between the spacing (9) and the communication channel (13) which is directly connected to the irrigation inlet duct (10), which is then connected to the irrigation hose (5).

The front base piece (2) fits, in a watertight way, into the cavity (16) in the rear base piece (3), which has a thread (12) used to connect the entire hydraulic system to a rod (19) that acts as an extension for moving the compact construction arrangement in a double lumen aspiration needle inside an "aspiration guide" (21) adapter device.

In particular, the compact design of the needle of this invention makes it possible to insert the entire assembly into the aspiration guide, as well as not using metal tubes internally.

In particular, the needles used by the present invention have an external diameter of approximately 3 mm, corresponding to the type of needle 12G with a length of 110 mm.

In a second embodiment, the present patent application deals with the use of the compact constructive configuration of the aspiration needle of the present invention in the collection of oocytes in animal ovaries, in an assisted reproduction procedure.

In general, in the procedure of oocyte collection and retrieval, an ultrasound follow-up of the process is used to guide the needle cannula to the correct position of the ovary. The needle bevel pierces the follicle and, to facilitate the detachment and flow of the oocytes, fluids are injected through the irrigation hose passing through the irrigation inlet duct that follows the communication channel, until it reaches the relief and communication space, where the injected fluid fills the entire space and begins to move through the spacing until it exits through the needle cannula. At this point, the silicone rubber sealing tube, which is pressed between the cavity and the aspiration hose, acts as a seal preventing the injected fluid from flowing back through the duct.

Next, the aspiration hose connected to a cap on a test tube, also connected with the same cap to a vacuum generation source (24) will aspiration the oocytes together with the fluid that is injected.

The technical advantage of using the compact design of the double lumen oocyte aspiration needle of this invention, in particular the application of the aspiration hose made of thermoplastic material with a low coefficient of friction and the compact and watertight design, is that this construction reduces by up to 40% the damage caused by friction between the oocyte body and the path taken during the aspiration, as is the case with double-lumen needles that allow contact with the aspirated oocytes with the metallic parts of the needle. Thus, the problem of obtaining viable and healthy oocytes is solved by using the present compact design of the aspiration needle of the present invention.

Also, the use of the front base piece accommodated with the rear base piece associated with the silicone rubber sealing tube eliminates the need to use long needle cannulas and long metal tubes.

### DETAILED DESCRIPTION OF THE INVENTION

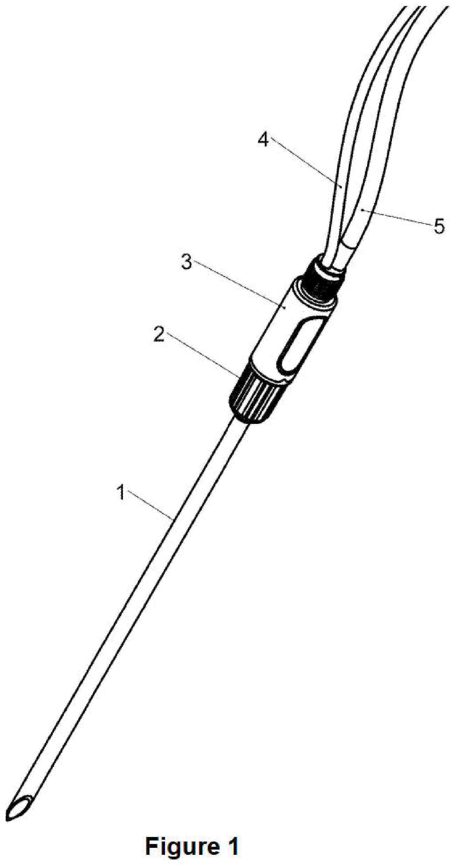

According to Figure 1 of this patent application, the compact design of the invention needle consists of an extraction hose (4) and an irrigation hose (5) connected to the bifurcated chamber, consisting of a rear base piece (3) coupled to a front base piece (2), and a needle (1).

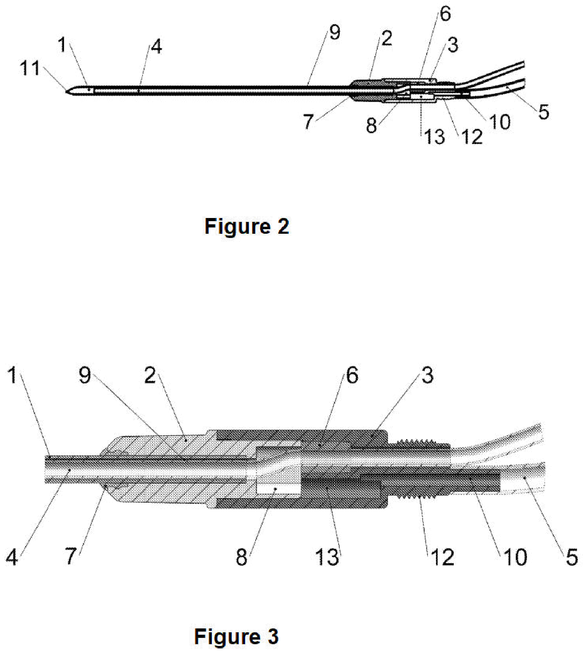

With regard to figure 2, the compact design of the aspiration needle with double lumen demonstrates the entire length of the aspiration hose (4), along the watertight hydraulic system, passing through the needle (1), front base part (2), relief and communication space (8), silicone rubber sealing tube (6), communication channel (13), rear base piece (3), coupling rod (12), irrigation inlet duct (10) and irrigation hose (5) .

Figure 3 shows all the details of the internal watertight hydraulic system of the compact construction configuration of the aspiration needle with double lumen of this invention, highlighting the arrangement of the bifurcated chamber, which is composed of the connection of the rear base part (3) coupled to a front base part (2) and its sealing parts, for example, the fastening area (7) with adhesion material, in particular, epoxy glue and the sealing tube (6) made of silicone rubber.

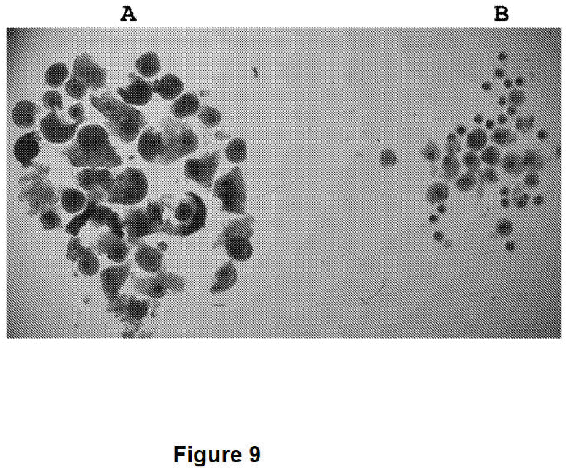

Figure 9 refers to a photograph taken by a Nikon SMZ800N stereoscope with a zoom ratio of 8:1 (zoom range of Ix-8x) and resolution of 640LP/mm (using Plan ED Apo 2x/WF objective at maximum zoom) with its alpha numerical aperture optics. Semi-apochromatic optics through a 4K alpha resolution digital camera of the PrimeCam model INTERVISION SMZ12, 12MP connected via cable to a computer with the Windows-based operating system, e.g. Windows 10, in which it presents the results of a comparative experiment in the quality of oocyte aspiration obtained, performed between a conventional double-lumen needle of metallic cannula with more than 500 mm (B) and with the compact constructive configuration of the needle of the invention (A) to verify the quality of viable oocytes. According to experimental results presented in (B), Figure 9 clearly shows the excessive wear of the cell walls of the oocytes aspirated by the conventional needle and the preservation of the aspirated oocytes by the compact constructive configuration of the aspiration needle with double lumen of the present invention, as indicated in the experimental results presented in (A).

Initially, the recovery of oocytes from four healthy quarter horse mares, from homogeneous genetic groups, with similar ages of 3 years, with the following weights, was evaluated: 512 kg for mare A, 533 kg for mare B, 522 kg for mare C and 499 for mare D.

In mares A and B, the 12G double-lumen aspiration needle of the present invention was used, while in mares C and D, the conventional 12G double-lumen long needle was used. Both were aspirated by vacuum pump model WTA-BV005d and the oocyte washing fluid used was the Dulbecco-modified saline phosphate buffer - DMPBS of the Reprodux brand and the composition is calcium chloride dihydrate - 0.133 g/1, magnesium chloride hexahydrate - 0.100g/l, potassium chloride - 0.200g/l, potassium phosphate monobasic - 0.200g/l, sodium chloride 8,000 g/1, sodium dibasic phosphate - 1,150 g/1, glucose - 1,000 g/1 and gentamicin - 0,050 g/1.

Throughout the aspiration process, the pressure used by all needles ranged from 120 mmHg to a maximum of 150 mmHg with a controlled temperature for the collection tube at 39°C.

After the collection of the oocytes, the aspirated material was transferred to a filter, where it was washed with the same solution used in aspiration, and sorted into two groups in a Petri dish for observation and analysis in a stereoscope SMZ800N Nikon with a zoom ratio of 8:1 (zoom range of Ix-8x) and resolution of 640LP/mm (using Plan ED Apo 2x / WE objective at maximum zoom) with its high magnification optics numerical aperture. Semi-apochromatic optics through a 12MP PrimeCam INTERVISION SMZ12 high-resolution 4K digital camera connected via cable to a Windows 10 computer. As shown in figure 9, the aspirated oocytes (right side) and identified as (B) were aspirated with a conventional 12G double-lumen needle and a metallic cannula with more than 500 mm and the aspirated oocytes (left side) and identified as (A) were aspirated with the 12G double-lumen aspiration needle of the present invention, where it is clear that the wear of the cells occurred in the oocytes on the right, due to the friction on the inner walls of the cannula of conventional long needles, on the left side, it is possible to notice a greater preservation of the cells.

### METHODOLOGY FOR FOLLICULAR ASPIRATION

Preparation of donor and recipient mares: donor mares were selected on the basis of their high genetic potential, even if they cannot conceive naturally, recipient mares are healthy and fertile females. Hormonal stimulation: The veterinarian specialized in equine reproduction performed hormonal stimulation in the donor and recipient mares through the application of progesterone, this stimulation aims to develop multiple ovarian follicles in the donor mares, where the oocytes are found. Use of ultrasound: The follicular aspiration procedure requires the use of ultrasound; the veterinarian used a modern ultrasound device of the brand Mindray with probe (transducer) model 65EC10EB adapted in a Sealed WTA aspiration guide with 8 mm mandrel to visualize the ovarian follicles in the donor mares. Follicular aspiration procedure: With the mare sedated and anesthetized, the veterinarian introduced the aspiration guide connected to an aspirator (WTA vacuum pump model BV005) through the vagina parallel to the cervix, using the images obtained by ultrasound, the professional can guide the aspiration guide to the ovarian follicle that contains the oocyte, an aspiration needle is aligned with the follicle and the oocyte is carefully aspirated. The procedures were performed on one or more follicles per individual, depending on the response to hormonal stimulation. Oocyte processing: the collected oocytes were washed, sorted, then processed in the laboratory to be analyzed and compared and analyzed for cell preservation.

## Claims

1. An OOCYTE ASPIRATION NEEDLE WITH DOUBLE LUMEN, **characterized in that** the oocyte aspiration needle comprises a needle (1) connected to a bifurcated chamber comprising a front base piece (2), a rear base piece(3), the front base piece (2) being coupled to a rear base piece (3), and a rod (19);
wherein said needle (1) has an aspiration hose (4) inserted internally into said needle (1);
wherein said aspiration hose (4) comprises a thermoplastic material with low friction coefficient; and
wherein said needle (1) has a total length less than 110 mm.

2. The NEEDLE according to claim 1, **characterized in that** the thermoplastic material of the aspiration hose (4) has a static friction coefficient of 0.05 p.

3. The NEEDLE according to claim 1, **characterized in that** said aspiration hose (4) is fastened and insulated, by means of a sealing tube (6) of silicone rubber, together with an adhesion material, to the entire internal watertight hydraulic system comprising the bifurcated chamber and said needle (1).

4. The NEEDLE according to claim 3, **characterized in that** said adhesion material comprises an epoxy glue.

5. The NEEDLE according to claim 1, **characterized in that** said needle (1) is fitted into a duct (17) and joined in the fastening region (7) with adhesion material wrapped in the housing (18) internal to said front base piece (2).

6. The NEEDLE according to claim 5, **characterized in that** said adhesion material comprises an epoxy glue.

7. The NEEDLE according to claim 1, **characterized in that** said aspiration hose (4) is passed through the channel (14) and kept pressed by the sealing tube (6) of silicone rubber housed in a cavity (15) subsequent and aligned with the channel (14).

8. The NEEDLE according to any of the claims 1 to 3, **characterized in that** said aspiration hose (4) is slightly curved in a space of relief and communication (8), through which it is directed internally to said needle (1) until close to the bevel (11), leaving a spacing (9) between the internal walls of said needle (1) and the external surface of said aspiration hose (4).

9. The NEEDLE according to claim 8, **characterized in that** said relief and communication space (8) creates a direct communication between said spacing (9) and the communication channel (13) which is directly connected to the irrigation inlet duct (10), which is connected to the irrigation hose (5).

10. The NEEDLE according to claim 1, **characterized in that** the front base piece (2) fits tightly to the cavity (16) in the rear base piece (3), and said rear base piece (3) has a rose (12) coupling the hydraulic system to a rod (19).

11. USE OF THE OOCYTE ASPIRATION NEEDLE WITH DOUBLE LUMEN, as defined in any of claims 1 to 10, **characterized in that** it is in the collection of oocytes in ovaries from animals, in assisted reproduction procedures.

12. The USE according to claim 11, **characterized in that** the animals comprise cattle, horses and/or pigs.
